# EUROPEAN PATENT APPLICATION

(11) **EP 4 678 624 A1**
(43) Date of publication of application: **14.01.2026**
(21) Application number: 24187201.9
(22) Date of filing: 08.07.2024
(51) Int. Cl.: C07C 45/67, C07C 47/277, C07C 47/263, C07C 47/24, C07C 49/203, C07C 67/293, C07C 69/14

(54) **DOUBLE BOND ISOMERIZATIONS USING COBALT DIMETHYLGLYOXIME CATALYSTS**

(71) Applicant: BASF SE, 67056 Ludwigshafen am Rhein (DE); University of Alberta, Edmonton, Alberta T6G 2R3 (CA)
(72) Inventor: RICHMOND, Edward, 67056 Ludwigshafen am Rhein (DE); SCHELWIES, Mathias, 67056 Ludwigshafen am Rhein (DE); PACIELLO, Rocco, 67098 Bad Duerkheim (DE); LIZANDARA PUEYO, Carlos, 67056 Ludwigshafen am Rhein (DE); LUNDGREN, Rylan, Edmonton, Alberta T6G 2G2 (CA); QIQIGE, Qiqige, Edmonton, Alberta T6G 2G2 (CA)
(74) Representative: Meissner Bolte Partnerschaft mbB

(57) **Abstract**

The present invention relates to a method for preparing a compound of the formula (I) 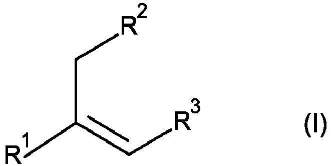 wherein R¹, R² and R³ are as defined in the claims and the description, by subjecting a compound of the formula (II) 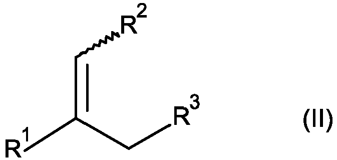 to a double-bond isomerization reaction in the presence of hydrogen and a cobalt dimethylglyoxime catalyst.

## Description

The present invention relates to a method for preparing a compound of the formula (I) 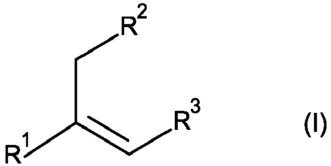 wherein R¹, R² and R³ are as defined below, by subjecting a compound of the formula (II) 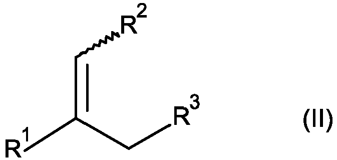 to a double-bond isomerization reaction in the presence of hydrogen and a cobalt dimethylglyoxime catalyst.

### TECHNICAL BACKGROUND

(E)-4-Acetoxy-2-methylbut-2-enal, also called C₅ acetate, is an important building block in industrial syntheses of retinol, stereoisomers and derivatives thereof (see e.g. G. L. Parker, L. K. Smith, I. R. Baxendale, Tetrahedron 2016, 72, 1645-1652).

Many synthetic routes towards the C₅ acetate yield first its methylene double bond isomer 3-formylbut-3-enyl acetate (i.e. the acetate of 4-hydroxy-2-methylene-butanal; 4-acetoxy-2-methylene-butyraldehyde) or a mixture of 3-formylbut-3-enyl acetate and the desired the C₅ acetate. For instance, the synthetic route starting from but-2-ene-1,4-diol described in US 4,124,619 via acetylation of the OH groups, hydroformylation at the C-C- double bond and elimination of acetic acid yields 3-formylbut-3-enyl acetate, which has to be subjected to a double bond isomerization to the desired C₅ acetate.

Also the photooxidation of isoprenyl acetate and simultaneous Kornblum-DeLaMare rearrangement described in WO2024/042132 and the photooxidation of isoprenyl acetate and transition metal-catalyzed rearrangement/dehydration described in WO2024/042131 yield mixtures of C₅ acetate and 3-formylbut-3-enyl acetate, where the latter, to obtain C₅ acetate, has to undergo a double bond isomerization.

The isomerization reaction described in US 4,124,619 is carried out under the action of a Pd catalyst, which is however rather costly. Moreover, the yield is not yet satisfactory.

CN 113233979 describes the C-C double bond isomerization of 3-formylbut-3-enyl acetate to 4-acetoxy-2-methylbut-2-enal using hydrogen in the presence of a catalyst system which contains Co(dmgBF₂)₂•L₂ (L is a solvent molecule) and a metal chloride selected from FeCl₂, FeCl₃, SnCl₂, SnCl₄, CuCI, CuCl₂ and ZnCl₂. The obtained conversion rates and selectivities are very good; the use of the metal chlorides is however linked with various disadvantages: An additional component has to be removed and if possible also recycled from the reaction mixture, making also the separation and recycling of the cobalt catalyst more complex. The presence of both catalyst (Co(dmgBF₂)₂•L₂) and metal chloride as co-catalyst increases in general the complexity of the system, especially if the reaction is to be carried out in continuous mode, and also the risk of negative catalyst/co-catalyst interactions. An important disadvantage is moreover the corrosivity of these metal chlorides, or chlorides in general, which can present a serious problem when upscaling the reaction, since reactions on industrial scale are mostly carried out in steel reactors. Furthermore, the applied hydrogen pressure of preferably 30 to 60 atm and 50 atm in the examples is rather high and also linked with higher costs. There is thus still room for improvement.

Many other double bond isomerization methods, as for example described in H. Wakamatsu, M. Nishida, N. Adachi, M. Mori J. Org. Chem. 2000, 65, 3966-3970, cannot be applied in this specific case since they lead to decomposition of the enal or to unselective isomerization.

Favorable conditions for double bond isomerizations are not only sought for the conversion of 3-formylbut-3-enyl acetate to 4-acetoxy-2-methylbut-2-enal, but also for other structures.

The object of the present invention was thus to provide a method for C-C double bond isomerizations which is widely applicable, i.e. not only for preparing 4-acetoxy-2-methylbut-2-enal from 3-formylbut-3-enyl acetate, but also for other systems, and which overcomes at least a part of the above disadvantages. Especially, the method should work under reaction conditions which are simpler and less demanding for both reactor (materials) and the reaction as such (allowing for example a simple(r) recycling of the catalyst and/or reducing the complexity of the reaction, especially if this is carried out in continuous mode) than the isomerisation method of CN 113233979, ideally without impairing product yield.

### SUMMARY OF THE INVENTION

This object is achieved by a method for preparing a compound of the formula (I) 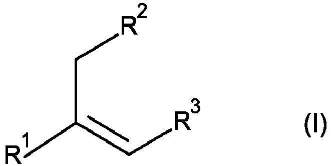 wherein
- R¹: is an electron-withdrawing group;
- R²: is hydrogen or C₁-C₆-alkyl; and
- R³: is selected from the group consisting of C₁-C₁₀-alkyl, C₁-C₁₀-haloalkyl, C₁-C₄-alkyl which carries a group R⁴, C₂-C₁₀-alkenyl, phenyl and -C(O)OR⁵; where
R⁴ is selected from the group consisting of -OR⁵, -O-C(O)-R⁵, -C(O)R⁵ and -C(O)OR⁵, and
each R⁵ is independently selected from the group consisting of hydrogen, C₁-C₄-alkyl, phenyl and benzyl;

or the electron-withdrawing group R¹ and R³, together with the carbon atoms they are bound to, form a 5-, 6- or 7-membered ring;
comprising subjecting a compound of the formula (II) 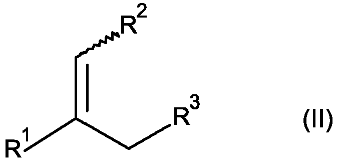
wherein R', R² and R³ are as defined above,
to a double-bond isomerization reaction in the presence of hydrogen and the cobalt complex Co(dmgX)₂•L₂ as catalyst, where X is H or BF₂ and L is a solvent molecule,
where the reaction is carried out under a pressure of from 1 to 10 bar (0.1 to 1 MPa).

Surprisingly, the reaction yields compounds (I) in excellent yields and selectivities at distinctly lower (hydrogen) pressures than the prior art methods, and does not require the presence of Fe, Sn, Cu or Zn chlorides (or more generally of Fe, Sn, Cu or Zn halides; actually it does not require any metal chlorides/halides with Lewis acid activity or any metal halide at all; or of any inorganic halide in general - except of course for the bridging ligand X (if this is BF₂) in the cobalt catalyst).

### DETAILED DESCRIPTION OF THE INVENTION

### Definitions

The wavy line in compounds (II) indicates that R² can be bound to point towards the same direction as R¹, or to point towards the same direction as -CH₂-R³. In case that R² is different from H, compound (II) can thus have E- or Z-configuration at the double bond carrying R¹, R² and -CH₂-R³, depending on the position of R² (and on the meanings of R¹ and R³, of course, which defines their priority according to the Cahn-Ingold-Prelog rules).

An electron-withdrawing group is an atom or group that draws electron density from neighboring atoms towards itself, usually by resonance (-M) or inductive (-I) effects. Typical examples are directly bound -C(=O)-containing groups, e.g. in the form of formyl (-CHO), alkylcarbonyl (-C(=O)-R, where R is alkyl) or alkoxycarbonyl (-C(=O)OR, where R is alkyl); halogenated alkyl groups, especially perhalogenated alkyl groups; such as CF₃; aryl, especially phenyl; and electron-poor heteroaromatic rings, such as pyridyl, pyridazinyl, pyrimidinyl, pyrazinyl or the triazines, where the electron-withdrawing effect of the aryl and electron-poor heteroaromatic rings can be further enhanced by suitable substituents on these rings, such as directly bound -C(=O)-containing groups or NO₂.

The term "halogen" denotes in each case fluorine, chlorine, bromine or iodine.

"Alkyl" is used in the usual sense. The term "alkyl" refers to saturated straight-chain (linear) or branched acyclic hydrocarbon radicals having, for example, 1 or 2 ("C₁-C₂-alkyl"), 1 to 4 ("C₁-C₄-alkyl"), 1 to 6 ("C₁-C₆-alkyl") or 1 to 10 ("C₁-C₁₀-alkyl") carbon atoms. C₁-C₂-Alkyl denotes a saturated linear or branched aliphatic acyclic hydrocarbon radical with 1 or 2 carbon atoms. Examples are methyl and ethyl. C₁-C₄-Alkyl denotes a saturated linear or branched aliphatic acyclic hydrocarbon radical with 1 to 4 carbon atoms. Examples are methyl, ethyl, n-propyl, isopropyl, n-butyl, sec-butyl, isobutyl and tert-butyl. C₁-C₆-Alkyl denotes a saturated linear or branched aliphatic acyclic hydrocarbon radical with 1 to 6 carbon atoms. Examples are, in addition to those mentioned for C₁-C₄-alkyl, n-pentyl, 1-methylbutyl, 2-methylbutyl, 3-methylbutyl, 2,2-dimethylpropyl, 1-ethylpropyl, 1,1-dimethylpropyl, 1,2-dimethylpropyl, n-hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, and (other) structural isomers thereof C₁-C₁₀-Alkyl denotes a saturated linear or branched aliphatic acyclic hydrocarbon radical with 1 to 10 carbon atoms. Examples are, in addition to those mentioned for C₁-C₆-alkyl, n-heptyl, n-octyl, 2-ethylhexyl, n-nonyl, n-decyl, 2-propylheptyl, and (other) structural isomers thereof.

The term "haloalkyl" (also expressed as "alkyl which is partially or fully halogenated") indicates saturated straight-chain or branched aliphatic non-cyclic hydrocarbon radicals having for example 1 to 2 ("C₁-C₂-haloalkyl") or 1 to 10 ("C₁-C₁₀-haloalkyl") carbon atoms, where some or all of the hydrogen atoms in these groups are replaced by halogen atoms as mentioned above, in particular fluorine, chlorine and/or bromine. "C₁-C₂-Haloalkyl" refers to alkyl groups having 1 or 2 carbon atoms (as mentioned above), where some or all of the hydrogen atoms in these groups are replaced by halogen atoms as mentioned above, in particular fluorine, chlorine and/or bromine. "C₁-C₁₀-Haloalkyl" refers to straight-chain or branched alkyl groups having 1 to 10 carbon atoms (as mentioned above), where some or all of the hydrogen atoms in these groups are replaced by halogen atoms as mentioned above, in particular fluorine, chlorine and/or bromine. Examples for C₁-C₂-haloalkyl are chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 2-chloroethyl, 1-bromoethyl, 2-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl or pentafluoroethyl. Examples for C₁-C₁₀-haloalkyl are, in addition to those mentioned for C₁-C₂-haloalkyl, 1-fluoropropyl, 2-fluoropropyl, 3-fluoropropyl, 1,1-difluoropropyl, 2,2-difluoropropyl, 1,2-difluoropropyl, 3,3-difluoropropyl, 3,3,3-trifluoropropyl, heptafluoropropyl, 1,1,1-trifluoroprop-2-yl, 1,1,1,3,3,3-hexafluoroprop-2-yl, heptafluoroprop-2-yl, 3-chloropropyl, 4-chlorobutyl and the like.

Strictly speaking, the term "alkenyl" indicates monounsaturated (i.e. containing one C-C double bond) straight-chain or branched aliphatic non-cyclic hydrocarbon radicals having for example 2 to 6 ("C₂-C₆-alkenyl") or 2 to 8 ("C₂-C₈-alkenyl") or 2 to 10 ("C₂-C₁₀-alkenyl") carbon atoms, where the C-C double bond can be in any position. As used in the present invention, the term encompasses however also "alkapolyenyl" groups, i.e. straight-chain or branched aliphatic non-cyclic hydrocarbon radicals having for example 4 to 6 ("C₄-C₆-alkapolyenyl") or 4 to 8 ("C₄-C₈-alkapolyenyl") or 4 to 10 ("C₄-C₁₀-alkapolyenyl") carbon atoms and two or more conjugated or isolated, but non-cumulated C-C double bonds. Examples for C₂-C₆-alkenyl in the strict sense (only 1 C-C double bond) are ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl, 1-ethyl-2-methyl-2-propenyl and the like. Examples for C₂-C₈-alkenyl in the strict sense (only 1 C-C double bond) are, in addition to the examples mentioned for C₂-C₆-alkenyl, 1-heptenyl, 2-heptenyl, 3-heptenyl, 4-heptenyl, 5-heptenyl, 6-heptenyl, 1-octenyl, 2-octenyl, 3-octenyl, 4-octenyl, 5-octenyl, 6-octenyl, 7-octenyl and the positional isomers thereof. Examples for C₂-C₁₀-alkenyl in the strict sense (only 1 C-C double bond) are, in addition to the examples mentioned for C₂-C₈-alkenyl, 1-nonenyl, 2-nonenyl, 3-nonenyl, 4-nonenyl, 5-nonenyl, 6-nonenyl, 7-nonenyl, 8-nonenyl, 1-decenyl, 2-decenyl, 3-decenyl, 4-decenyl, 5-decenyl, 6-decenyl, 7-decenyl, 8-decenyl, 9-decenyl and the positional isomers thereof.

Examples for alkapolyenyl groups are buta-1,3-dien-1-yl, buta-1,3-dien-2-yl, penta-1,3-dien-1-yl, penta-1,3-dien-2-yl, penta-1,3-dien-3-yl, penta-1,3-dien-4-yl, penta-1,3-dien-5-yl, penta-1,4-dien-1-yl, penta-1,4-dien-2-yl, penta-1,4-dien-3-yl, and the like.

To avoid any doubt, if R¹ and R³, together with the carbon atoms they are bound to, form a 5-, 6- or 7-membered ring, this ring encompasses an electron-withdrawing group, this being directly bound to the carbon atom of the scaffold carrying R¹.

Formyl is a group -C(=O)H (also simply designated as -CHO).

To avoid any doubt, C(O) designates a carbonyl group C(=O). Thus, -C(O)OH is a carboxyl group -C(=O)OH, and -C(O)OR is an ester group -C(=O)OR.

C₁-C₂-alkylcarbonyl is a group -C(=O)R, where R is C₁-C₂-alkyl (i.e. methyl or ethyl). Examples are acetyl and propionyl.

C₁-C₃-alkanols are methanol, ethanol, n-propanol or isopropanol.

"dmg" denotes the ligand dimethylglyoxime (HO-N=C(CH₃)-C(CH₃)=N-OH). In the present cobalt complexes, dmg acts as a bidentate ligand, binding to Co via its nitrogen atoms. The solvent molecules L are bound as axial ligands. In solid state, the Co(dmgX)₂•L₂ complex is assumed to have following structure:

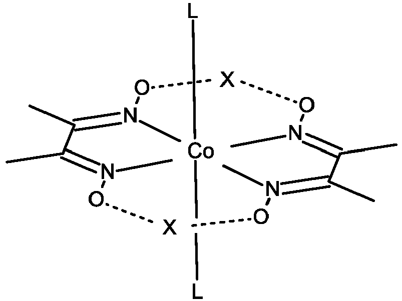

Co(dmgX)₂•L₂ depicts the catalyst in its starting form. In the course of the reaction, the catalyst is of course modified, e.g. by the presence of hydrogen. Without wishing to be bound by theory, it is assumed that during the catalytic cycle, hydride (actually a hydrogen radical) coordinates to the central metal, thus formally converting Co(ll) into Co(III). The hydrogen radical is transferred to compound (II), where it adds to the C-C double bond; and Co(lll) is formally reduced again to Co(II). Abstraction of a hydrogen radical from the methylene group carrying R³ yields the compound (I); and the abstracted hydrogen radical coordinates again to the central metal, converting this again formally to Co(lll) and restarting the catalytic cycle. The double-bond isomerization reaction being carried out "in the presence of Co(dmgX)₂•L₂ as catalyst" does thus of course not mean that Co(dmgX)₂•L₂ remains unchanged during the reaction, but that it is inserted in this form into the reaction and ideally, after completion of the reaction, is again present in this form.

### Embodiments (E.x) of the invention

General and preferred embodiments E.x are summarized in the following, non-exhaustive list. Further preferred embodiments become apparent from the paragraphs following this list.

E.1. A method for preparing a compound of the formula (I) 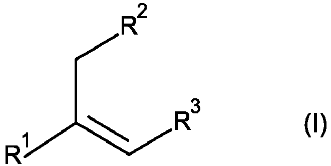 wherein
- R¹: is an electron-withdrawing group;
- R²: is hydrogen or C₁-C₆-alkyl; and
- R³: is selected from the group consisting of C₁-C₁₀-alkyl, C₁-C₁₀-haloalkyl, C₁-C₄-alkyl which carries a group R⁴, C₂-C₁₀-alkenyl, phenyl and -C(O)OR⁵;
where
- R⁴: is selected from the group consisting of -OR⁵, -O-C(O)-R⁵, -C(O)R⁵ and -C(O)OR⁵; and
- each R⁵: is independently selected from the group consisting of hydrogen, C₁-C₄-alkyl, phenyl and benzyl;

or the electron-withdrawing group R¹ and R³, together with the carbon atoms they are bound to, form a 5-, 6- or 7-membered ring;
comprising subjecting a compound of the formula (II) 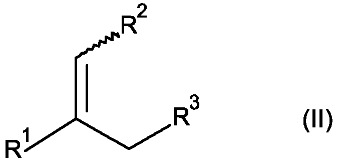
wherein R', R² and R³ are as defined above,
to a double-bond isomerization reaction in the presence of hydrogen and Co(dmgX)₂•L₂ as catalyst, where X is H or BF₂ and L is a solvent molecule,
where the reaction is carried out under a pressure of from 1 to 10 bar (0.1 to 1 MPa).

E.2. The method according to embodiment E.1, where the reaction is carried out in the absence of any Sn, Fe, Cu or Zn chloride.

E.3. The method according to embodiment E.2, where the reaction is carried out in the absence of any metal chloride with Lewis acid activity.

E.4. The method according to embodiment E.3, where the reaction is carried out in the absence of any metal chloride.

E.5. The method according to any of the preceding embodiments, where the reaction mixture contains less than 0.02 mol%, preferably less than 0.01 mol%, in particular less than 0.005 mol% of Sn, Fe, Cu and Zn chlorides, relative to the amount of compound (II) (the amount as introduced into the reaction, of course).

E.6. The method according to embodiment E.5, where the reaction mixture contains less than 0.02 mol%, preferably less than 0.01 mol%, in particular less than 0.005 mol% of any metal chloride with Lewis acid activity, relative to the amount of compound (II) (the amount as introduced into the reaction, of course).

E.7. The method according to embodiment E.6, where the reaction mixture contains less than 0.02 mol%, more preferably less than 0.01 mol%, in particular less than 0.005 mol% of any chloride, relative to the amount of compound (II) (the amount as introduced into the reaction, of course).

E.8. The method according to any of the preceding embodiments, where R¹ is selected from the group consisting of formyl (-CHO), C₁-C₂-alkylcarbonyl, -C(O)OH, -C(O)OR⁶, where R⁶ is C₁-C₄-alkyl; and phenyl.

E.9. The method according to embodiment E.8, where R¹ is selected from the group consisting of from formyl, -C(O)OH and phenyl.

E.10. The method according to any of the preceding embodiments, where R² is hydrogen or C₁-C₄-alkyl.

E.11. The method according to embodiment E.10, where R² is hydrogen.

E.12. The method according to any of the preceding embodiments, where R³ is selected from the group consisting of C₁-C₂-haloalkyl, C₁-C₂-alkyl which carries a group R⁴, C₂-C₈-alkenyl, -C(O)OR⁵ and phenyl; where R⁴ is selected from the group consisting of -OR⁵, -O-C(O)-R⁵, and -C(O)R⁵; and where each R⁵ is independently selected from the group consisting of hydrogen, C₁-C₂-alkyl and benzyl.

E.13. The method according to embodiment E.12, where R⁴ is selected from the group consisting of -OR^{5a}, -O-C(O)-R^{5b}, and -C(O)R^{5c}; where R^{5a} is selected from the group consisting of hydrogen, C₁-C₂-alkyl and benzyl; R^{5b} is C₁-C₂-alkyl; and R^{5c} is C₁-C₂-alkyl.

E.14. The method according to any of embodiments E.1 to E.7, E.10 and E.11, where R¹ and R³ form together a bridging group *-C(O)-(CH₂)ₙ-^{#}, where n is 2 or 3, or a bridging group *-C(O)-O-(CH₂)ₘ-^{#}, where m is 1 or 2; where * denotes the attachment point of R¹ and ^{#} denotes the attachment point of R³.

E.15. The method according to any of the preceding embodiments, where in compounds (I) and (II) R¹ is -CHO, R² is H and R³ is -CH₂-O-C(O)-R⁵, where R⁵ is C₁-C₄-alkyl, preferably C₁-C₂-alkyl.

E.16. The method according to any of the preceding embodiments, where in compounds (I) and (II):
R¹ is -CHO, R² is H and R³ is -CH₂-O-C(O)-CH₃; or
R¹ is -CHO, R² is H and R³ is -CH₂-OH; or
R¹ is -CHO, R² is H and R³ is -CH₂-OCH₃; or
R¹ is -CHO, R² is H and R³ is -CH₂-O-benzyl; or
R¹ is -CHO, R² is H and R³ is -CH₂CH₂-C(O)-CH₃; or
R¹ is -CHO, R² is H and R³ is -CH₂CH₂Cl; or
R¹ is -CHO, R² is H and R³ is -(CH₂)₆CH=CH₂; or
R¹ is -CHO, R² is H and R³ is -CH₂-CH=C(CH₃)₂; or
R¹ is -CHO, R² is H and R³ is phenyl; or
R¹ is phenyl, R² is H and R³ is -CH₂-O-C(O)-CH₃; or
R¹ is -C(O)OH, R² is H and R³ is -C(O)OCH₃; or
R² is H, and R¹ and R³ form together a bridging group *-C(O)-(CH₂)₃-^{#}, where * denotes the attachment point of R¹ and ^{#} denotes the attachment point of R³; or
R² is n-butyl, and R¹ and R³ form together a bridging group *-C(O)-(CH₂)₂-^{#},
where * denotes the attachment point of R¹ and ^{#} denotes the attachment point of R³; or
R² is H, and R¹ and R³ form together a bridging group *-C(O)-O-CH₂-^{#}, where * denotes the attachment point of R¹ and ^{#} denotes the attachment point of R³.

E.17. The method according to embodiment E.16, where R¹ is -CHO, R² is H and R³ is -CH₂-O-C(O)-CH₃.

E.18. The method according to any of the preceding embodiments, where X is BF₂.

E.19. The method according to any of embodiments E.1 to E.17, where X is H.

E.20. The method according to any of the preceding embodiments, where the reaction is carried out under a pressure of from 1 to 9 bar (0.1 to 0.9 MPa).

E.21. The method according to embodiment E.20, where the reaction is carried out under a pressure of from 1 to 7 bar (0.1 to 0.7 MPa).

E.22. The method according to embodiment E.21, where the reaction is carried out under a pressure of from 4 to 7 bar (0.4 to 0.7 MPa).

E.23. The method according to embodiment E.22, where the reaction is carried out under a pressure of from 5 to 7 bar (0.5 to 0.7 MPa).

E.24. The method according to any of the preceding embodiments, where L is selected from the group consisting of water, C₁-C₃-alkanols, acetonitrile, tetrahydrofuran and N,N-dimethylformamide molecules.

E.25. The method according to embodiment E.24, where L is a water molecule.

E.26. The method according to any of the preceding embodiments, where the reaction is carried out at from 20 to 100°C.

E.27. The method according to embodiment E.26, where the reaction is carried out at from 25 to 100°C.

E.28. The method according to embodiment E.27, where the reaction is carried out at from 30 to 90°C.

E.29. The method according to embodiment E.28, where the reaction is carried out at from 30 to 80°C, e.g. from 35 to 80°C.

E.30. The method according to embodiment E.29, where the reaction is carried out at from 30 to 60°C, e.g. from 35 to 60°C.

E.31. The method according to embodiment E.30, where the reaction is carried out at from 30 to 50°C, e.g. from 35 to 50°C.

E.32. The method according to any of the preceding embodiments, where X is BF₂ and the reaction is carried out at from 30 to 80°C, preferably from 30 to 60°C or 35 to 60°C; more preferably from 30 to 50°C or 35 to 50°C.

E.33. The method according to any of embodiments E.1 to E.31, where X is H and the reaction is carried out at from 35 to 100°C, preferably from 40 to 90°C, more preferably from 50 to 90°C, in particular from 60 to 90°C, specifically from 75 to 85°C.

E.34. The method according to any of the preceding embodiments, where the reaction is carried out in an organic solvent.

E.35. The method according to embodiment E.34, where the solvent is selected from the group consisting of aromatic hydrocarbons, such as benzene, toluene, the xylenes, chlorobenzene or dichlorobenzene; acetonitrile, open-chain ethers, such as dimethyl ether, diethyl ether, dipropyl ether, diisopropyl ether or methyl-tert-butyl ether; cyclic ethers, such as tetrahydrofuran, 2-methyltetrahydrofuran or 1,4-dioxane; and mixtures of the aforementioned solvents.

E.36. The method according to embodiment E.35, where the solvent is selected from the group consisting of toluene, the xylenes, acetonitrile, tetrahydrofuran, 2-methyltetrahydrofuran and mixtures thereof.

E.37. The method according to any of the preceding embodiments, where the catalyst is used in an amount of 0.01 to 10 mol-%, relative to the amount of compound (II).

E.38. The method according to embodiment E.37, where the catalyst is used in an amount of 0.1 to 10 mol-%, relative to the amount of compound (II).

E.39. The method according to embodiment E.38, where the catalyst is used in an amount of 0.5 to 10 mol-%, relative to the amount of compound (II).

E.40. The method according to embodiment E.39, where the catalyst is used in an amount of 0.5 to 7 mol-%, relative to the amount of compound (II).

E.41. The method according to embodiment E.40, where the catalyst is used in an amount of 0.5 to 5 mol-%, relative to the amount of compound (II).

The isomerization reaction can be depicted as follows:

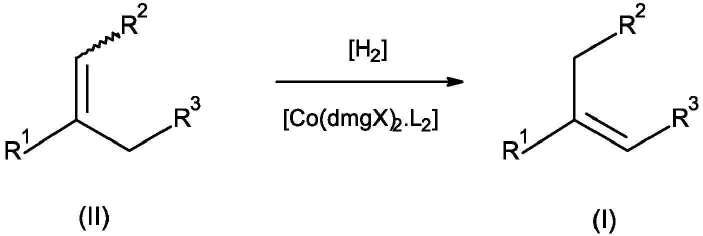

The method of the invention generally affords compounds (I) in high selectivity in the shown configuration, i.e. with R¹ and R³ being predominantly in E-position to each other; therefore, and also to allow easier reading, only this molecule geometry is shown above and wherever compounds (I) are depicted within the present application. Compounds (I) wherein R¹ and R³ are in Z-position to each other are formed, if at all, in amounts of at most 25 mol-%, preferably <10 mol-%, more preferably <5 mol-%, relative to the total amount of the E- and Z-isomers of compounds (I) (E- and Z-isomers relating in this context only to the configuration of the C-C double bond shown above).

As already said, surprisingly, the reaction yields compounds (I) in excellent yields and selectivities at distinctly lower hydrogen pressures or pressure in general than the prior art methods, and does not require the presence of Fe, Sn, Cu or Zn chlorides (or more generally of any Fe, Sn, Cu or Zn halides); actually it does not require any metal chlorides/halides with Lewis acid activity, or any halide at all, be it in the form of metal halides or of hydrohalic acids, such as HCl (except of course for the bridging ligand X (if this is BF₂) in the cobalt catalyst).

Thus, preferably, the reaction is carried out in the absence of any Sn, Fe, Cu and Zn chloride (or any Sn, Fe, Cu and Zn halide in general); in particular in the absence of any metal chloride (or halide in general) with Lewis acid activity. To even better avoid corrosion, the reaction is more preferably carried out in the absence of any chloride (or halide in general), be it in the form of metal chlorides (or metal halides in general) or of hydrochloric acids (or hydrohalic acids in general) (except of course for the bridging ligand X (if this is BF₂) in the cobalt catalyst).

Metal chlorides with Lewis acid activity are, for example, in addition to the above-mentioned Fe, Sn, Cu and Zn chlorides, chlorides of metals of groups 3 to 15 (including lanthanides and actinides) of the periodic table of elements (the group numbering relating to the IUPAC nomenclature of 1985). More generally, metal halides with Lewis acid activity are, for example, in addition to the above-mentioned Fe, Sn, Cu and Zn chlorides, halides of metals of groups 3 to 15 (including lanthanides and actinides) of the periodic table of elements (the group numbering relating to the IUPAC nomenclature of 1985).

Alternatively expressed, the reaction mixture (generally comprising or consisting of compound (II), the catalyst, one or more solvents, if any, optionally an analytical standard (e.g. N-methylpyrrolidone as NMR standard) and after the start of the reaction also hydrogen, compound (I) and by-products, if any) does preferably not contain any intentionally added Sn, Fe, Cu and Zn chlorides and more preferably does not contain any intentionally added metal chloride with Lewis acid activity. "Intentionally added" means to reflect that such chlorides may be contained unintentionally as impurities in the reaction mixture. If unintentionally present, they are however contained in neglectable amounts. Thus, the reaction mixture preferably contains less than 0.02 mol%, more preferably less than 0.01 mol%, in particular less than 0.005 mol% of Sn, Fe, Cu and Zn chlorides, relative to the amount of compound (II) (the amount as introduced into the reaction, of course). In particular, the reaction mixture contains less than 0.02 mol%, more preferably less than 0.01 mol%, e.g. less than 0.005 mol% of any metal chloride with Lewis acid activity, relative to the amount of compound (II) (the amount as introduced into the reaction, of course). More particularly, the reaction mixture contains less than 0.02 mol%, more preferably less than 0.01 mol%, e.g. less than 0.005 mol% of any chloride, relative to the amount of compound (II) (the amount as introduced into the reaction, of course).

In compounds (I) and (II), R¹, R² and R³ generally have the same meaning. The below remarks to preferred meanings of these radicals therefore apply both to starting compound (II) and to product (I).

R¹ is preferably selected from the group consisting of formyl (-CHO), C₁-C₂-alkylcarbonyl, -C(O)OH, -C(O)OR⁶, where R⁶ is C₁-C₄-alkyl; and phenyl; more preferably from formyl, -C(O)OH and phenyl.

R² is preferably hydrogen or C₁-C₄-alkyl, and is more preferably hydrogen.

R³ is preferably selected from the group consisting of C₁-C₂-haloalkyl, C₁-C₂-alkyl which carries a group R⁴, C₂-C₈-alkenyl, -C(O)OR⁵ and phenyl; where R⁴ is selected from the group consisting of -OR⁵, -O-C(O)-R⁵, and -C(O)R⁵; and where each R⁵ is independently selected from the group consisting of hydrogen, C₁-C₂-alkyl and benzyl. More preferably, R⁴ is selected from the group consisting of -OR^{5a}, -O-C(O)-R^{5b}, and -C(O)R^{5c}; where R^{5a} is selected from the group consisting of hydrogen, C₁-C₂-alkyl and benzyl; R^{5b} is C₁-C₂-alkyl; and R^{5c} is C₁-C₂-alkyl.

In an alternatively preferred embodiment, R¹ and R³ form together a bridging group *-C(O)-(CH₂)ₙ-^{#}, where n is 2 or 3, or a bridging group *-C(O)-O-(CH₂)ₘ-^{#}, where m is 1 or 2; where * denotes the attachment point of R¹ and ^{#} denotes the attachment point of R3.

In a particularly preferred embodiment, R¹ is -CHO, R² is H and R³ is -CH₂-O-C(O)-R⁵, where R⁵ is C₁-C₄-alkyl, preferably C₁-C₂-alkyl.

Specifically, in compounds (I) and (II):
- R¹ is -CHO, R² is H and R³ is -CH₂-O-C(O)-CH₃; or
- R¹ is -CHO, R² is H and R³ is -CH₂-OH; or
- R¹ is -CHO, R² is H and R³ is -CH₂-OCH₃; or
- R¹ is -CHO, R² is H and R³ is -CH₂-O-benzyl; or
- R¹ is -CHO, R² is H and R³ is -CH₂CH₂-C(O)-CH₃; or
- R¹ is -CHO, R² is H and R³ is -CH₂CH₂Cl; or
- R¹ is -CHO, R² is H and R³ is -(CH₂)₆CH=CH₂; or
- R¹ is -CHO, R² is H and R³ is -CH₂-CH=C(CH₃)₂; or
- R¹ is -CHO, R² is H and R³ is phenyl; or
- R¹ is phenyl, R² is H and R³ is -CH₂-O-C(O)-CH₃; or
- R¹ is -C(O)OH, R² is H and R³ is -C(O)OCH₃; or
- R² is H, and R¹ and R³ form together a bridging group *-C(O)-(CH₂)₃-^{#}, where * denotes the attachment point of R¹ and ^{#} denotes the attachment point of R³; or
- R² is n-butyl, and R¹ and R³ form together a bridging group *-C(O)-(CH₂)₂-^{#}, where * denotes the attachment point of R¹ and ^{#} denotes the attachment point of R³; or
- R² is H, and R¹ and R³ form together a bridging group *-C(O)-O-CH₂-^{#}, where * denotes the attachment point of R¹ and ^{#} denotes the attachment point of R³.

Very specifically, in compounds (I) and (II) R¹ is -CHO, R² is H and R³ is -CH₂-O-C(O)-CH₃; i.e. compound (I) is 4-acetoxy-2-methylbut-2-enal, preferably (E)-4-acetoxy-2-methylbut-2-enal, also called C₅ acetate, and compound (II) is 3-formylbut-3-enyl acetate.

In a preferred embodiment of the catalyst Co(dmgX)₂•L₂, X is BF₂.

In another preferred embodiment, X is H.

In a preferred embodiment of the catalyst Co(dmgX)₂•L₂, L is selected from the group consisting of water, C₁-C₃-alkanols (i.e. methanol, ethanol, n-propanol or isopropanol), acetonitrile, tetrahydrofuran and N,N-dimethylformamide molecules, preferably from of water, methanol, acetonitrile, tetrahydrofuran and N,N-dimethylformamide; and is more preferably a water molecule.

In a more preferred embodiment, X is BF₂ and L is a water molecule. In another preferred embodiment, X is H and L is a water molecule.

Preferably, the catalyst is used in an amount of 0.01 to 10 mol-%, more preferably 0.1 to 10 mol-%, even more preferably 0.5 to 7 mol-%, in particular 0.5 to 5 mol-%, relative to the amount of compound (II)) (the amount as introduced into the reaction, of course).

The reaction is preferably carried out in an organic solvent. Suitable organic solvents are for example aromatic hydrocarbons, such as benzene, toluene, the xylenes, chlorobenzene or dichlorobenzene; acetonitrile; open-chained ethers, such as dimethyl ether, diethyl ether, dipropyl ether, diisopropyl ether or methyl-tert-butyl ether; cyclic ethers, such as tetrahydrofuran, 2-methyltetrahydrofuran or 1,4-dioxane; and mixtures of the aforementioned solvents. Among the above solvents, preference is given to aromatic hydrocarbons, acetonitrile, cyclic ethers and mixtures thereof. In particular, the solvent is selected from the group consisting of toluene, the xylenes, acetonitrile, tetrahydrofuran, 2-methyltetrahydrofuran and mixtures thereof. Among these, for an easier separation and recycling of the catalyst, preference is given to toluene, the xylenes, acetonitrile, 2-methyltetrahydrofuran and mixtures thereof.

The reaction is preferably carried out under a pressure of from 1 to 9 bar (0.1 to 0.9 MPa), more preferably from 1 to 7 bar (0.1 to 0.7 MPa), even more preferably from 4 to 7 bar (0.4 to 0.7 MPa), and specifically from 5 to 7 bar (0.5 to 0.7 MPa). The pressure is exerted by hydrogen or by mixtures of hydrogen and nitrogen. Suitable hydrogen/nitrogen mixtures contain 20 to 95 vol.-% of hydrogen, preferably 30 to 80 vol.-% of hydrogen, relative to the total volume of hydrogen and nitrogen. Preferably, however, the pressure is exerted by hydrogen; in this case, the pressure values relate to hydrogen pressure. If a mixture of hydrogen and nitrogen is used, the pressure values relate to the pressure exerted with this mixture.

The quality (purity) of the hydrogen gas is not very critical; preference is however given to a purity of ≥99%, more preferably ≥99.5%. If hydrogen is used in admixture with nitrogen, the quality (purity) of the nitrogen gas is not very critical, either; preference is however given to a purity of ≥99%, more preferably ≥99.5%.

The reaction is preferably carried out in a temperature range of from 20 to 100°C, more preferably from 25 to 100°C, even more preferably from 30 to 90°C, in particular from 30 to 80°C, e.g. from 35 to 80°C; or from 30 to 60°C, e.g. from 35 to 60°C; or from 30 to 50°C, e.g. from 35 to 50°C.

The optimum temperature depends on various factors and can be easily determined by the skilled person. The optimum reaction temperature depends inter alia on the catalyst used, especially on the meaning of X. To be more precise, catalysts wherein X is H are somewhat less active than catalysts wherein X is BF₂, and thus higher temperatures are more advantageous in this case.

Accordingly, in a preferred embodiment, X is BF₂ and the reaction is carried out at from 30 to 80°C, preferably from 30 to 60°C, e.g. from 35 to 60°C; more preferably from 30 to 50°C, e.g. from 35 to 50°C.

In another preferred embodiment, X is H and the reaction is carried out at from 35 to 100°C, preferably from 40 to 90°C, more preferably from 50 to 90°C, in particular from 60 to 90°C, specifically from 75 to 85°C.

The reaction is generally carried out by mixing compound (II) with the catalyst, expediently in a solvent, inserting hydrogen up to the desired pressure and bringing the reaction mixture to the desired temperature, or inversely, first bringing the reaction mixture to the desired temperature and then inserting hydrogen up to the desired pressure; the first variant being preferred. To replace all or at least the major part of ambient gas by hydrogen, it is expedient to charge hydrogen under pressure (to speed up exchange of atmosphere, this may exceed the reaction pressure) and again release the pressure once or several times before the proper start of the reaction, i.e. before the hydrogen charged to and kept at the desired pressure and the reaction mixture is brought to the desired temperature. The reaction mixture is expediently stirred during reaction.

The reaction can be carried out in any reactor known in the art as suitable for reactions under pressure. Suitable reactors contain at least a means for introducing and releasing hydrogen and contain expediently also a stirrer and means for cooling or heating. Suitable standard reactors for gas-liquid reaction systems are known to those skilled in the art and are described, for example, in Ullmann's Encyclopedia of Industrial Chemistry, 2005, Wiley-VCH Verlag GmbH & Co. KGaA, chapter 3.3, Reactor Types and Their Industrial Applications and Reactors for Gas-Liquid Reactions. Examples are pressure-resistant stirred tanks, which are usually also referred to as autoclaves.

After completion of the reaction, the pressure is released, expediently after cooling (if the reaction temperature has exceeded ambient temperature), and the reaction mixture is worked up to remove the catalyst and, if desired, to isolate and purify the product (I). Work-up and isolation/purification of the compound (I) can be carried out by usual means, such as extraction, distillation or chromatographic methods.

The method of the invention is widely applicable and yields compounds (I) in excellent yields and selectivities at distinctly lower hydrogen pressures than the prior art methods. It does not require the presence of Fe, Sn, Cu or Zn chlorides (or more generally of Fe, Sn, Cu or Zn halides); actually it does not require any metal chlorides/halides with Lewis acid activity at all, and thus allows to reduce production costs, since the reaction system is less complex both from processing and work-up view (the latter because said chlorides/halides need not be separated off and the cobalt catalyst can be separated and recycled without the additional step of separating the same from the co-catalyst), negative catalyst/co-catalyst interactions are precluded and corrosion is avoided or at least reduced, especially if steel reactors are used.

The following examples serve as further illustration of the invention.

### EXAMPLES

### Abbreviations

- Ac: acetyl (-C(O)CH₃)
- Me: methyl
- Bn: benzyl (-CH₂-C₆H₅)
- Ph: phenyl

Unless noted otherwise, all reactions were set up in a N₂-filled glovebox. All glassware was oven-dried prior to use. Parr high pressure reactors (Series 4750 vessels with split ring closure) were used for high pressure reactions.

Unless noted, yields and conversions were determined by quantitative ¹H NMR with N-methylpyrrolidone (NMP) as the internal standard.

Flash chromatography was performed as described by Still and co-workers (Still, W. C.; Kahn, M.; Mitra, A., J. Org. Chem. 1978, 43, 2923-2925; SiliaFlash P60, 40-63µm, 60Å silica gel, Silicycle) or by automated flash chromatography (Isolera, HP-SIL silica cartridges, Biotage).

Analytical thin-layer chromatography was performed using glass plates pre-coated with silica (SiliaPlate G TLC - Glass-Backed, 250µm, Silicycle). TLC plates were visualized by UV light and/or staining with aqueous basic potassium permanganate.

HRMS analyses of the products were performed on an Agilent Technologies 6220 oaTOF instrument (ESI, APPI, APCI) in positive or negative ionization mode or a Kratos MS50G Double Focussing Sector (EB/EI) instrument in positive mode.

NMR spectra (¹H, ¹³C, ¹⁹F) were obtained on an Agilent VNMRS 700 MHz, Varian VNMRS 600 MHz, Varian VNMRS 500 MHz, or Varian 400 MHz spectrometer. The chemical shifts are given as parts per million (ppm) and were referenced to the residual solvent signal (CDCl₃: δH = 7.26 ppm, δC = 77.06 ppm).

Co(dmgBF₂)₂•2H₂O and Co(dmgH)₂•2H₂O were synthesized using the procedure according to Li, G.; Han, A.; Pulling, M. E.; Estes, D. P.; Norton, J. R., J. Am. Chem. Soc. 2012, 134, 14662-14665. The ligands were purchased from Sigma-Aldrich, Strem Chemicals, Combi-blocks, Alfa Aesar, or Acros.

The substrates (ll.x) were obtained from commercial vendors and used as supplied.

The substrates are moreover available by following literature procedures:
II.2, II.3, II.6, II.7: Peed, J.; Davies, !. R.; Peacock, L. R.; Taylor, J. E.; Kociok-Köhn, G.; Bull, S. D., J. Org. Chem. 2012, 77, 543-555
II.4: (a) Hurtley, A.E., Lu, Z. and Yoon, T.P., Angew. Chem. Int. Ed., 2014, 53, 8991-8994. (b) Batt, F.; Bourcet, E.; Kassab, Y.; Fache, F. Synlett 2007, 1869-1872. (c) Hughes, R. C.; Dvorak, C. A.; Meyers, A. I., J. Org. Chem. 2001, 66, 5545-5551.
II.5: (a) Douelle, F.; Capes, A. S.; Greaney, M. F. Org. Lett., 2007, 9, 1931-34. (b) Larsen, M. A.; Hennessy, E. T.; Deem, M. C.; Lam, Y.-h.; Sauri, J.; Sather, A. C., J. Am. Chem. Soc. 2020, 142, 726-732
II.8: Al Hazmi, A. M.; Sheikh, N. S.; Bataille, C. J. R.; Al-Hadedi, A. A. M.; Watkin, S. V.; Luker, T. J.; Camp, N. P.; Brown, R. C. D., Org. Lett. 2014, 16, 5104-5107
II.9: Erkkilä, A. and Pihko, P.M., Eur. J. Org. Chem. 2007, 4205-4216
II.10: Bugarin, A.; Jones, K. D.; Connell, B. T., Chem. Commun. 2010, 46, 1715-1717
!!.11: Li, J.; Zhu, Y.; Lu, Y.; Wang, Y.; Liu, Y.; Liu, D.; Zhang, W., Organometallics 2019, 38, 3970-3978
II.12: Song, L.; Yao, H.; Zhu, L.; Tong, R., Org. Lett. 2013, 15, 6-9
II.13: Shu, C.; Mega, R. S.; Andreassen, B. J.; Noble, A.; Aggarwal, V. K., Angew. Chem. Int. Ed., 2018, 57, 15430-15434

### General Procedure

In a glovebox filled with N₂, Co(dmgBF₂)₂•2H₂O (0.0125 mmol, 5.3 mg) was added in a 2-dram vial followed by the respective substrate (compound Il.x) (0.5 mmol) and N-methyl-2-pyrrolidone (5-10 mg) as internal NMR standard. Toluene (5.0 mL) and a stir-bar was added to the mixture, the vial was sealed with a PTFE-line cap pierced with an 18-gauge needle, then the vial was placed into a high-pressure reactor. The high-pressure reactor was sealed and taken out of the glovebox. The N₂ atmosphere of the reactor was replaced by H₂ by charging to 3-10 bar (0.3-1 MPa), then carefully releasing the pressure, this process was repeated two more times. The reaction mixture was stirred under corresponding H₂ pressure (5.1 to 6.8 bar (0.51 to 0.68 MPa) at 50°C for two hours. Upon completion of the reaction, the reactor was cooled to room temperature, and H₂ was released. The reaction vial was taken out from the reactor and the crude yield was determined using ¹H NMR by removing a small aliquot (~10 µL) of the reaction mixture and diluting with CDCl₃. Reported terminal NMR yields were obtained in a similar manner. The product was then purified by silica gel chromatography by loading the reaction mixture directly onto the column.

### Example 1

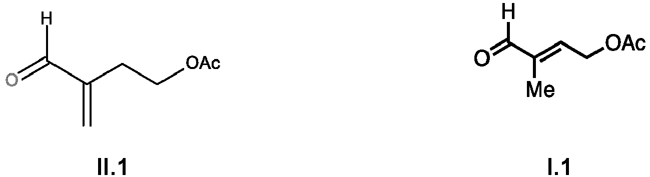

Compound 1.1 was prepared according to the General Procedure from the corresponding enal II.1 (71.0 mg, 0.50 mmol), 50°C, 2 h. ¹H NMR conversion: 90%, crude yield: 90%. Isolated in 96% yield as a mixture of product and substrate (91:9 I.1/II.1) and as a colourless oil after purification by silica column chromatography.

Analytical data for I.1/II.1 (91:9):
¹H NMR (CDCl₃, 500 MHz, I.1) δ 9.45 (s, 1H), 6.49 (tq, *J* = 6.0, 1.4 Hz, 1H), 4.89 (dq, *J* = 6.0, 1.1 Hz, 2H), 2.12 (s, 3H), 1.79 (d, *J* = 1.1 Hz, 3H);
¹H NMR (CDCl₃, 500 MHz, residual !!.1) δ 9.54 (s, 1H), 6.34 (s, 1H), 6.10 (s, 1H), 4.18 (t, *J* = 6.6 Hz, 2H), 2.59 (td, *J* = 6.6, 1.1 Hz, 2H), 2.02 (s, 3H);
¹³C NMR (CDCl₃, 125 MHz, 1.1) δ 194.1, 170.6, 145.7, 140.6, 60.9, 20.8, 9.5;
HRMS (ESI): calcd. for C₇H₁₀O₃Na [M+Na]⁺ 165.0522. Found 165.0521.

### Example 2

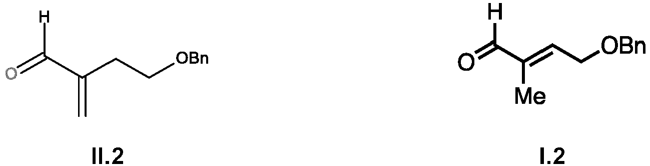

Compound I.2 was prepared according to the General Procedure from the corresponding enal II.2 (95.0 mg, 0.50 mmol), 50°C, 2 h. ¹H NMR conversion: 94%, crude yield: 94%. Isolated in 90% yield as a colourless oil after purification by silica column chromatography (25% pentane in diethyl ether).

Analytical data for I.2:
¹H NMR (CDCl₃, 500 MHz) δ 9.45 (s, 1H), 7.39-7.31 (M, 5H), 6.61 (tq, *J* = 5.6, 1.3 Hz, 1H), 4.59 (S, 2H), 4.36 (dq, *J* = 5.6, 1.1 Hz, 2H), 1.73 (s, 3H);
¹³C NMR (CDCl₃, 125 MHz) δ 194.5, 149.5, 139.5, 137.5, 128.6, 128.1, 127.9, 73.2, 66.8, 9.6;
HRMS (ESI): calcd. for C₁₂H₁₄O₂Na [M+Na]⁺ 213.0886. Found 213.0885.

### Example 3

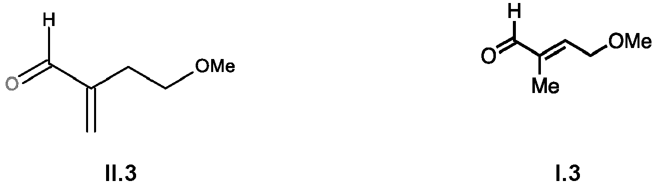

Compound I.3 was prepared according to the General Procedure from the corresponding enal II.3 (57.0 mg, 0.50 mmol), 50°C, 2 h. ¹H NMR conversion: 94%, crude yield: 81%. Isolated in 84% yield as a colourless oil after purification by silica column chromatography (25% pentane in diethyl ether).

Analytical data for I.3:
¹H NMR (CDCl₃, 500 MHz) δ 9.44 (s, 1H), 6.54 (tq, *J* = 5.6, 1.2 Hz, 1H), 4.27 (dq, *J* = 5.6, 1.2 Hz, 2H), 3.41 (s, 3H), 1.75 (q, *J* = 1.2 Hz, 3H);
¹³C NMR (CDCl₃, 125 MHz) δ 194.4, 149.4, 139.5, 69.3, 58.8, 9.5;
HRMS (ESI): calcd. for C₆H₁₀O₂Na [M+Na]⁺ 137.0573. Found 137.0573.

### Example 4

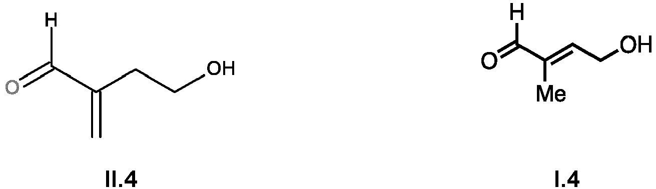

Compound I.4 was prepared according to the General Procedure from the corresponding enal II.4 (100.1 mg, 1.00 mmol), 50°C, 2 h. ¹H NMR conversion: 98%, crude yield: 90%. Isolated in 90% yield as a colourless oil after purification by silica column chromatography (30% pentane in diethyl ether).

Analytical data for I.4:
¹H NMR (CDCl₃, 500 MHz) δ 9.44 (s, 1H), 6.60 (tq, *J* = 5.7, 1.3 Hz, 1H), 4.57-4.53 (m, 2H), 2.00 (brs, 1H), 1.77 (s, 3H);
¹³C NMR (CDCl₃, 125 MHz) δ 194.7, 151.6, 138.8, 59.8, 9.5;
HRMS (ESI): calcd. for C₅H₈O₂Na [M+Na]⁺ 123.0417. Found 123.0418.

### Example 5

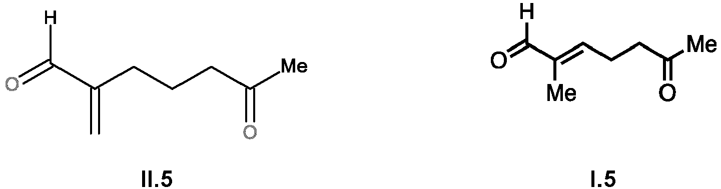

Compound I.5 was prepared according to the General Procedure from the corresponding enal II.5 (35.0 mg, 0.25 mmol), 50°C, 2 h. ¹H NMR conversion: >99%, crude yield: >98%, 92:8 E/Z ratio. Isolated in 88% yield as a mixture of geometric isomers (92:8 E/Z) and as a colourless oil after purification by silica column chromatography (5% pentane in diethyl ether).

Analytical data for I.5:
¹H NMR (CDCl₃, 500 MHz, major, E-product) δ 9.37 (s, 1H), 6.42 (tq, *J* = 7.0, 1.4 Hz, 1H), 2.67-2.64 (m, 2H), 2.62-2.57 (m, 2H), 2.18 (s, 3H), 1.76 (d, *J* = 1.4 Hz, 3H);
¹H NMR (CDCl₃, 500 MHz, minor, Z-product) δ 9.47 (s, 1H), 6.74 (tq, *J* = 6.9, 1.4 Hz, 1H), 3.51 (d, *J* = 7.0Hz, 2H), 2.67-2.64 (m, 2H), 2.25 (s, 3H), 1.75 (s, 3H);
¹³C NMR (CDCl₃, 125 MHz, major, E-prod) δ 206.8, 195.1, 152.4, 140.1, 41.8, 29.9, 23.0, 9.2;
HRMS (ESI): calcd. for C₈H₁₂O₂Na [M+Na]⁺ 163.0730. Found 163.0729.

### Example 6

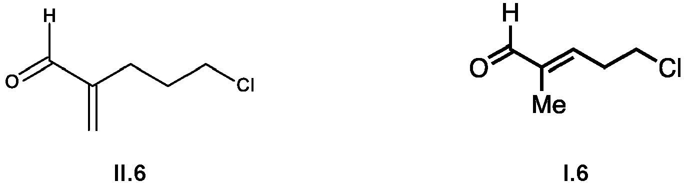

Compound I.6 was prepared according to the General Procedure from the corresponding enal II.6 (66.5 mg, 0.50 mmol), 50°C, 2 h. ¹H NMR conversion: >99%, crude yield: 91%. Isolated in 81% yield as a colourless oil after purification by silica column chromatography (20% pentane in diethyl ether).

Analytical data for I.6:
¹H NMR (CDCl₃, 500 MHz) δ 9.45 (s, 1H), 6.51 (tq, *J* = 7.3, 1.4 Hz, 1H), 3.67 (t, *J* = 6.7 Hz, 2H), 2.83 (dt, *J* = 7.3, 6.7 Hz, 2H), 1.78 (s, 3H);
¹³C NMR (CDCl₃, 125 MHz) δ 194.8, 184.7, 141.4, 42.6, 31.9, 9.5;
HRMS (ESI): calcd. for C₆H₉ONa [M+Na]⁺ 155.0234. Found 155.0232.

### Example 7

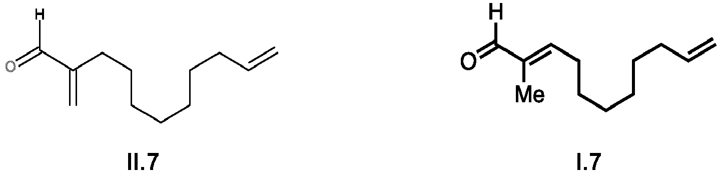

Compound I.7 was prepared according to the General Procedure from the corresponding enal II.7 (90.0 mg, 0.50 mmol), 50°C, 2 h. ¹H NMR conversion: >99%, crude yield: 90%. Isolated in 89% yield as a colourless oil after purification by silica column chromatography (5% pentane in diethyl ether).

Analytical data for I.7:
¹H NMR (CDCl₃, 500 MHz) δ 9.40 (s, 1H), 6.49 (tq, *J* = 7.5, 1.3 Hz, 1H), 5.85-5.76 (m, 1H), 5.02-4.92 (m, 2H), 2.35 (td, *J* = 7.3, 7.3 Hz, 2H), 2.05 (td, *J* = 7.5, 6.7 Hz, 2H), 1.74 (s, 3H), 1.54-1.48 (m, 2H), 1.42-1.32 (m, 6H);
¹³C NMR (CDCl₃, 125 MHz) δ 195.4, 155.0, 139.4, 139.0, 114.4, 33.7, 29.2, 29.0, 28.9, 28.8, 28.4, 9.2;
HRMS (ESI): calcd. for C₁₂H₂₀ONa [M+Na]⁺ 203.1406. Found 203.1402.

### Example 8

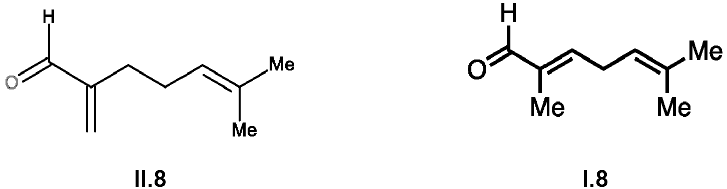

Compound I.8 was prepared according to the General Procedure from the corresponding enal II.8 (69.0 mg, 0.50 mmol), 50°C, 2 h. ¹H NMR conversion: >99%, crude yield: 90%. Isolated in 90% yield as a colourless oil after purification by silica column chromatography (5% pentane in diethyl ether).

Analytical data for I.8:
¹H NMR (CDCl₃, 500 MHz) δ 9.39 (s, 1H), 6.43 (tq, *J* = 6.7, 1.3 Hz, 1H), 5.16 (t, *J* = 7.2 Hz, 1H), 3.03 (dd, *J* = 7.2, 6.7 Hz, 2H), 1.77 (s, 3H), 1.73 (s, 3H), 1.67 (s, 3H);
¹³C NMR (CDCl₃, 125 MHz) δ 195.4, 153.2, 138.9, 134.6, 119.3, 28.2, 25.7, 17.9, 9.2;
HRMS (ESI): calcd. for C₉H₁₄ONa [M+Na]⁺ 161.0937. Found 161.0937.

### Example 9

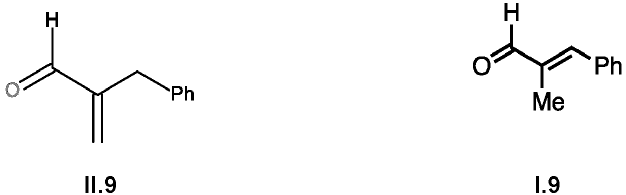

Compound I.9 was prepared according to the General Procedure from the corresponding enal II.9 (73.0 mg, 0.50 mmol), 50°C, 2 h. ¹H NMR conversion: >99%, crude yield: 98%. Isolated in 93% yield as a colourless oil after purification by silica column chromatography (5% pentane in diethyl ether).

Analytical data for I.9:
¹H NMR (CDCl₃, 500 MHz) δ 9.60 (s, 1H), 7.55-7.53 (m, 2H), 7.47-7.44 (m, 2H), 7.42-7.40 (m, 1H), 7.27 (d, *J* = 1.1 Hz, 1H), 2.09 (d, *J* = 1.1 Hz, 3H);
¹³C NMR (CDCl₃, 125 MHz) δ 195.6, 149.8, 138.4, 135.2, 130.1, 129.6, 128.7, 11.0;
HRMS (ESI): calcd. for C₁₀H₁₀ONa [M+Na]⁺ 169.0624. Found 169.0625.

### Example 10

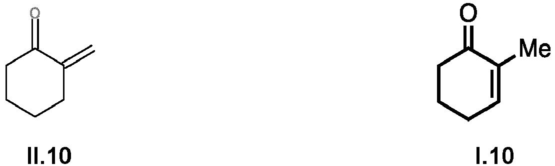

Compound !.10 was prepared according to the General Procedure from the corresponding enone II.10 (11.0 mg, 0.10 mmol), 50°C, 2 h. ¹H NMR conversion: >99%, crude yield: >98%. Spectroscopic data for the product agreed with the literature (Gradillas, A., Belmonte, E., da Silva, R.F. and Pérez-Castells, J., Eur. J. Org. Chem., 2014: 1935-1941)

### Example 11

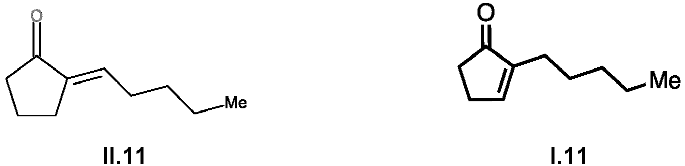

Compound !.11 was prepared according to the General Procedure from the corresponding enone !!.11 (76.0 mg, 0.50 mmol), 50°C, 2 h. ¹H NMR conversion: >99%, crude yield: 85%. Isolated in 82% yield as a colourless oil after purification by silica column chromatography (5% pentane in diethyl ether).

Analytical data for !.11:
¹H NMR (CDCl₃, 500 MHz) δ 7.30-7.28 (m, 1H), 2.57-2.54 (m, 2H), 2.40-2.38 (m, 2H), 2.18-2.13 (m, 2H), 1.50-1.44 (m, 2H), 1.34-1.26 (m, 4H), 0.89-0.87 (m, 3H);
¹³C NMR (CDCl₃, 125 MHz) δ 210.1, 157.3, 146.6, 34.7, 31.6, 27.5, 26.5, 24.8, 22.5, 14.0;
HRMS (ESI): calcd. for C₁₀H₁₆ONa [M+Na]⁺ 175.1093. Found 175.1090.

### Example 12

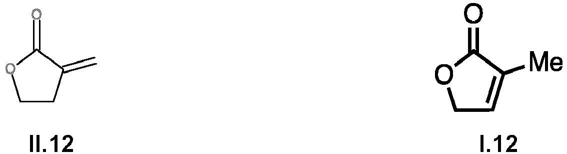

Compound 1.12 was prepared according to the General Procedure from the corresponding lactone II.12 (49.0 mg, 0.50 mmol), 50°C, 2 h. ¹H NMR conversion: >99%, crude yield: 98%. Isolated in 94% yield as a colourless oil after purification by silica column chromatography (25% pentane in diethyl ether).

Analytical data for !.12:
¹H NMR (CDCl₃, 500 MHz) δ 7.14-7.12 (m, 1H), 4.76-4.74 (m, 2H), 1.94-1.92 (m, 3H);
¹³C NMR (CDCl₃, 125 MHz) δ 174.8, 144.9, 130.0, 70.0, 10.7;
HRMS (ESI): calcd. for C₅H₆O₂Na [M+Na]⁺ 121.0260. Found 121.0260.

### Example 13

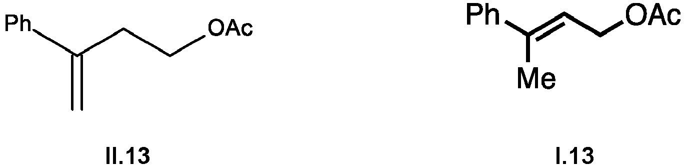

Compound 1.13 was prepared according to the General Procedure from the corresponding substrate II.13 (34.2 mg, 0.18 mmol), 50°C, 2 h. ¹H NMR conversion: 86%, crude yield: 86%, 76:24 E/Z ratio. Isolated in 90% yield as a mixture of geometric isomers (77:18:5 *E*/*Z*/II.13) as a colourless oil after purification by silica column chromatography (10% pentane in diethyl ether).

Analytical data for !.13:
¹H NMR (CDCl₃, 500 MHz, major, E-product) δ 7.42-7.40 (m, 2H), 7.35-7.32 (m, 2H), 7.29-7.26 (m, 1H), 5.90 (tq, *J* = 7.0, 1.4 Hz, 1H), 4.79 (d, *J* = 7.0 Hz, 2H), 2.12 (s, 3H), 2.09 (s, 3H);
¹H NMR (CDCl₃, 500 MHz, minor, Z-product) δ 7.35-7.26 (m, 3H), 7.19-7.17 (m, 2H), 5.66 (tq, *J* = 7.2, 1.5 Hz, 1H), 4.50 (dd, *J* = 7.2, 0.9 Hz, 2H), 2.11 (d, *J* = 0.9 Hz, 3H), 2.05 (s, 3H);
¹³C NMR (CDCl₃, 125 MHz, major, E-product) δ 171.1, 142.6, 140.2, 128.3, 127.6, 125.9, 121.4, 61.7, 21.1, 16.2;
HRMS (ESI): calcd. for C₁₂H₁₄O₂Na [M+Na]⁺ 213.0886. Found 213.0883.

### Example 14

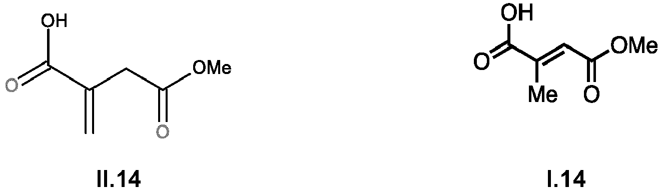

Compound 1.14 was prepared according to the General Procedure from itaconic acid monomethyl ester II.14 (14.4 mg, 0.10 mmol), 80°C, 5 h. ¹H NMR conversion: 79%, crude yield: 75%. Isolated in 68% yield as a white solid after purification by silica column chromatography (10% hexane in EtOAc).

Analytical data for I.14:
¹H NMR (CDCl₃, 500 MHz) δ 6.89 (q, *J* = 1.6 Hz, 1H), 3.15 (s, 3H), 2.30 (d, *J* = 1.6 Hz, 3H);
¹³C NMR (CDCl₃, 125 MHz) δ 171.4, 166.1, 142.7, 128.3, 51.9, 14.0;
HRMS (ESI): calcd. for C₆H₇O₄ [M-H]⁻ 143.0350. Found 143.0351.

### Example 15

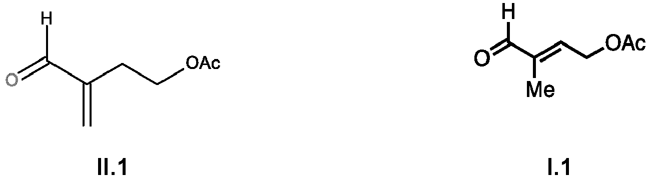

The experiment of example 1 was repeated; however with following variations: The catalyst was used in an amount of 5 mol%, relative to the amount of compound !!.1 (instead of the 2.5 mol% used in example 1), the temperature was 35°C, the solvent was acetonitrile and the pressure was 5.1 bar (0.51 MPa). ¹H NMR conversion: 93%, yield: 80%.

### Example 16

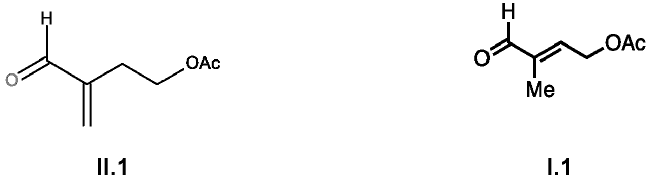

The experiment of example 15 was repeated; however with following variations: The catalyst was Co(dmgH)₂•2H₂O, the temperature was 80°C, the reaction time was 5 hours, and the solvent was tetrahydrofuran (THF). ¹H NMR conversion: 90%, yield: 83%.

### Example 17

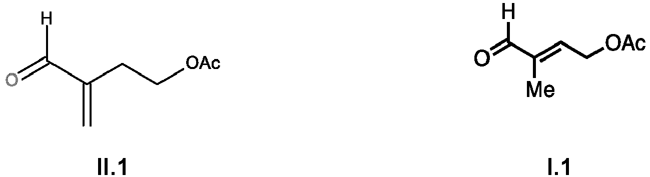

The experiment of example 15 was repeated; however with following variations: The solvent was toluene. ¹H NMR conversion: 94%, yield: 84%.

### Example 18

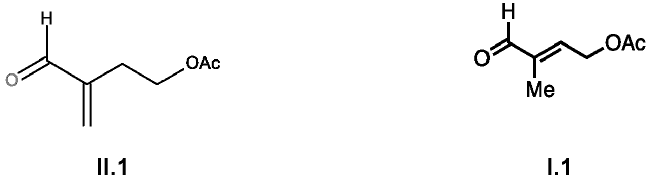

The experiment of example 15 was repeated; however with following variations: The catalyst was used in an amount of 0.5 mol%, relative to the amount of compound II.1 (instead of the 5 mol% used in example 15), the temperature was 80°C, the reaction time was 24 h, and the solvent was toluene. ¹H NMR conversion: 91%, yield: 77%.

### Example 19

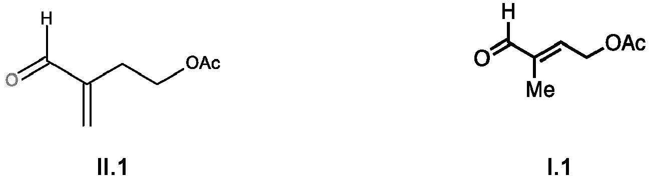

The experiment of example 1 was repeated; however on a 0.1 mmol scale and with a hydrogen pressure of 5.1 bar (0.51 MPa). ¹H NMR conversion: 94%, yield: 86%.

Comparative example Comp.-19:
The experiment of example 19 was repeated; however, 0.25% by weight of SnCl₂, relative to the amount of substrate II.1, were additionally added to the reaction mixture. ¹H NMR conversion: 91%, yield: 76%.

As can be seen, conversion and yield in example 19 according to the invention are not impaired by the absence of SnCl₂. To the contrary, and unexpectedly, conversion and yield are even somewhat higher than in the otherwise analogous reaction of Comp.-19, however additionally carried out in the presence of SnCl₂.

### Comparative example 20

To allow a better comparison with the prior art method, example 5 of CN 113233979 was repeated (1 wt.% of Co(dmgBF₂)₂•2H₂O, relative to the weight of the substrate !!.1, 0.1 wt.% of SnCl₂, relative to the weight of the substrate II.1, 50°C, 51 bar (50 atm; 5.1 MPa) hydrogen pressure, 10 h); however on a 1 mmol scale to allow a better comparability with the present examples. ¹H NMR conversion: 95%, yield: 85% of !.1; 15% of II.1.

As can be seen, conversion and yield are in the same range as the present reactions of compound !!.1 to 1.1. This shows that the present, milder, reaction conditions are not to the expense of conversion rate and yield.

## Claims

1. A method for preparing a compound of the formula (I) 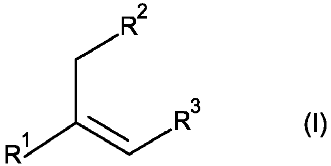 wherein
R¹ is an electron-withdrawing group;
R² is hydrogen or C₁-C₆-alkyl; and
R³ is selected from the group consisting of C₁-C₁₀-alkyl, C₁-C₁₀-haloalkyl, C₁-C₄-alkyl which carries a group R⁴, C₂-C₁₀-alkenyl, phenyl and -C(O)OR⁵;
where
R⁴ is selected from the group consisting of -OR⁵, -O-C(O)-R⁵, -C(O)R⁵ and -C(O)OR⁵; and
each R⁵ is independently selected from the group consisting of hydrogen, C₁-C₄-alkyl, phenyl and benzyl;
or the electron-withdrawing group R¹ and R³, together with the carbon atoms they are bound to, form a 5-, 6- or 7-membered ring;
comprising subjecting a compound of the formula (II) 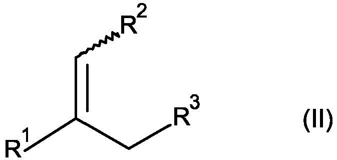
wherein R', R² and R³ are as defined above,
to a double-bond isomerization reaction in the presence of hydrogen and Co(dmgX)₂•L₂ as catalyst, where X is H or BF₂ and L is a solvent molecule,
where the reaction is carried out under a pressure of 1 to 10 bar (0.1 to 1 MPa).

2. The method according to claim 1, where the reaction is carried out in the absence of any Sn, Fe, Cu or Zn chloride; in particular in the absence of any metal chloride with Lewis acid activity.

3. The method according to any of the preceding claims, where R¹ is selected from the group consisting of formyl (-CHO), C₁-C₂-alkylcarbonyl, -C(O)OH, -C(O)OR⁶, where R⁶ is C₁-C₄-alkyl; and phenyl; preferably from formyl, -C(O)OH and phenyl.

4. The method according to any of the preceding claims, where R² is hydrogen or C₁-C₄-alkyl, preferably hydrogen.

5. The method according to any of the preceding claims, where R³ is selected from the group consisting of C₁-C₂-haloalkyl, C₁-C₂-alkyl which carries a group R⁴, C₂-Cs-alkenyl, -C(O)OR⁵ and phenyl; where R⁴ is selected from the group consisting of -OR⁵, -O-C(O)-R⁵, and -C(O)R⁵; where R⁵ is selected from the group consisting of hydrogen, C₁-C₂-alkyl and benzyl;
where preferably R⁴ is selected from the group consisting of -OR^{5a}, -O-C(O)-R^{5b}, and -C(O)R^{5c}; where R^{5a} is selected from the group consisting of hydrogen, C₁-C₂-alkyl and benzyl; R^{5b} is C₁-C₂-alkyl; and R^{5c} is C₁-C₂-alkyl.

6. The method according to any of claims 1, 2 or 4, where R¹ and R³ form together a bridging group *-C(O)-(CH₂)ₙ-^{#}, where n is 2 or 3, or a bridging group *-C(O)-O-(CH₂)ₘ-^{#}, where m is 1 or 2; where * denotes the attachment point of R¹ and^{#} denotes the attachment point of R³.

7. The method according to any of the preceding claims, where in compounds (I) and (II) R¹ is -CHO, R² is H and R³ is -CH₂-O-C(O)-R⁵, where R⁵ is C₁-C₄-alkyl.

8. The method according to any of claims 1 to 6, where in compounds (I) and (II):
- R¹ is -CHO, R² is H and R³ is -CH₂-O-C(O)-CH₃; or
- R¹ is -CHO, R² is H and R³ is -CH₂-OH; or
- R¹ is -CHO, R² is H and R³ is -CH₂-OCH₃; or
- R¹ is -CHO, R² is H and R³ is -CH₂-O-benzyl; or
- R¹ is -CHO, R² is H and R³ is -CH₂CH₂-C(O)-CH₃; or
- R¹ is -CHO, R² is H and R³ is -CH₂CH₂Cl; or
- R¹ is -CHO, R² is H and R³ is -(CH₂)₆CH=CH₂; or
- R¹ is -CHO, R² is H and R³ is -CH₂-CH=C(CH₃)₂; or
- R¹ is -CHO, R² is H and R³ is phenyl; or
- R¹ is phenyl, R² is H and R³ is -CH₂-O-C(O)-CH₃; or
- R¹ is -C(O)OH, R² is H and R³ is -C(O)OCH₃; or
- R² is H, and R¹ and R³ form together a bridging group *-C(O)-(CH₂)₃-^{#}, where * denotes the attachment point of R¹ and ^{#} denotes the attachment point of R³; or
- R² is n-butyl, and R¹ and R³ form together a bridging group *-C(O)-(CH₂)₂-^{#}, where * denotes the attachment point of R¹ and ^{#} denotes the attachment point of R³; or
- R² is H, and R¹ and R³ form together a bridging group *-C(O)-O-CH₂-^{#}, where * denotes the attachment point of R¹ and ^{#} denotes the attachment point of R3.

9. The method according to any of the preceding claims, where the reaction is carried out under a pressure of from 1 to 9 bar (0.1 to 0.9 MPa), preferably from 1 to 7 bar (0.1 to 0.7 MPa), more preferably from 4 to 7 bar (0.4 to 0.7 MPa), specifically from 5 to 7 bar (0.5 to 0.7 MPa).

10. The method according to any of the preceding claims, where X is BF₂.

11. The method according to any of the preceding claims, where L is selected from the group consisting of water, C₁-C₃-alkanols, acetonitrile, tetrahydrofuran and N,N-dimethylformamide molecules, and is preferably a water molecule.

12. The method according to any of the preceding claims, where the reaction is carried out at from 20 to 100°C, preferably from 25 to 100°C, more preferably from 30 to 90°C, specifically from 35 to 80°C or from 35 to 60°C or from 30 to 50°C.

13. The method according to any of the preceding claims, where the reaction is carried out in an organic solvent.

14. The method according to claim 13, where the solvent is selected from the group consisting of aromatic hydrocarbons, such as benzene, toluene, the xylenes, chlorobenzene or dichlorobenzene; acetonitrile; open-chained ethers, such as dimethyl ether, diethyl ether, dipropyl ether, diisopropyl ether or methyl-tert-butyl ether; cyclic ethers, such as tetrahydrofuran, 2-methyltetrahydrofuran or 1,4-dioxane; and mixtures of the aforementioned solvents, and is preferably selected from the group consisting of toluene, the xylenes, acetonitrile, tetrahydrofuran, 2-methyltetrahydrofuran and mixtures thereof.

15. The method according to any of the preceding claims, where the catalyst is used in an amount of 0.01 to 10 mol-%, preferably 0.5 to 7 mol-%, in particular 0.5 to 5 mol-%, relative to the amount of compound (II).
